# EUROPÄISCHE PATENTSCHRIFT

(11) **EP 4 426 364 B1**
(45) Veröffentlichungstag und Bekanntmachung des Hinweises auf die Patenterteilung: **18.03.2026**
(21) Anmeldenummer: 22813965.5
(22) Anmeldetag: 04.11.2022
(51) Int. Cl.: A61L 29/04, A61L 29/06, A61L 29/10, A61L 29/14

(54) **MEDIZINPRODUKT UND VERFAHREN ZUR HERSTELLUNG EINES PRODUKTS, INSBESONDERE MEDIZINPRODUKTS**
MEDICAL PRODUCT AND METHOD FOR PRODUCING A PRODUCT, IN PARTICULAR A MEDICAL PRODUCT
PRODUIT MÉDICAL ET PROCÉDÉ DE FABRICATION D'UN PRODUIT, EN PARTICULIER D'UN PRODUIT MÉDICAL

(30) Priorität: 04.11.2021 DE 102021212452; 08.04.2022 DE 102022203586
(43) Veröffentlichungstag der Anmeldung: 11.09.2024
(73) Patentinhaber: B. Braun Melsungen AG, 34212 Melsungen (DE)
(72) Erfinder: SITTKUS, Benjamin, 78166 Donaueschingen, Pfohren (DE)
(74) Vertreter: Patentanwälte Ruff, Wilhelm, Beier, Dauster & Partner mbB
(86) Internationale Anmeldenummer: PCT/EP2022/080818
(87) Internationale Veröffentlichungsnummer: WO 2023/079076

(56) Entgegenhaltungen:
- US-A1- 2003 028 210
- US-A1- 2017 119 450
- LO SHAO-HSIANG ET AL: "A Wireless Parylene-Based Cardiovascular Pressure Sensor with Mxene Film", 2019 20TH INTERNATIONAL CONFERENCE ON SOLID-STATE SENSORS, ACTUATORS AND MICROSYSTEMS & EUROSENSORS XXXIII (TRANSDUCERS & EUROSENSORS XXXIII), IEEE, 23 June 2019 (2019-06-23), pages 2231 - 2234, XP033599930, DOI: 10.1109/TRANSDUCERS.2019.8808336
- CHEN LONGLONG ET AL: "High-Tactile Sensitivity of Piezoresistive Sensors With a Micro-Crack Structure Induced by Thin Film Tension", IEEE ELECTRON DEVICE LETTERS, IEEE, USA, vol. 40, no. 9, 1 September 2019 (2019-09-01), pages 1519 - 1521, XP011742094, ISSN: 0741-3106, [retrieved on 20190822], DOI: 10.1109/LED.2019.2927720

## Beschreibung

### ANWENDUNGSGEBIET UND STAND DER TECHNIK

Die Erfindung betrifft ein Medizinprodukt und ein Verfahren zum Herstellen eines Medizinprodukts .

Aufgrund der hohen und weiter steigenden Inzidenz von arteriosklerotisch bedingten Erkrankungen sind fortgeschrittene intraoperative Diagnostik- sowie Therapieansätze grundsätzlich von großer gesellschaftlicher und wirtschaftlicher Bedeutung. Eine Digitalisierung von regulierten medizinischen Anwendungen erfordert eine gesicherte Datenbasis und damit sich ergänzende und unabhängige Verfahren zur Gewinnung von Informationen. Hierzu tragen unterschiedliche und manchmal redundante Sensoren bei. Bislang eingesetzte Verfahren bei arteriosklerotisch bedingten Erkrankungen, wie beispielsweise bildgebenden Verfahren (Kontrastmittel-Angiographie, Doppler sowie intravaskulärer Ultraschall) zur Schweregrad-Beurteilung von pathologischen Gefäßveränderungen, sind dabei auf die Gefäßdurchmesser-Analyse und somit den Stenosegrad begrenzt. Die Therapieentscheidung kann durch eine feinere Differenzierung verbessert werden, wozu jedoch zusätzliche Informationen über mechanische Gefäßwandeigenschaften notwendig sind. Während moderne und komplexe intravaskuläre Ultraschall-Systeme insbesondere in Studien zum Einsatz kommen, um Einblicke in die lokale Gefäßstruktur zu erhalten, sind einfache Sensorlösungen, welche in der klinischen Routine Anwendung finden und insbesondere die Informationslücke über mechanische Gewebseigenschaften schließen könnten, bisher nicht am Markt erhältlich.

Bekannt ist ein implantierbarer, drahtloser Sensor zur Überwachung des Blutdrucks, wobei der Sensor ein flexibles Substrat aus Parylene, eine Kupferschicht, eine Chrom/Gold-Schicht sowie eine PDMS-Schicht aufweist (Lo Shao-Hsiang et al.: "A Wireless Parylene-Based Cardiovascular Pressure Sensor with Mxene Film", 2019 20TH INTERNATIONAL CONFERENCE ON SOLID-STATE SENSORS, ACTUATORS AND MICROSYSTEMS & EUROSENSORS XXXIII (TRANSDUCERS & EUROSENSORS XXXIII)).

Aus der US 2003/028210 A1 ist ein metallischer Ballonkatheter bekannt, dessen Ballon mit einer Beschichtung aus einem biokompatiblen Elastomer versehen sein kann, um den Ballon im Falle einer Metallermüdung und/oder einer Rissbildung vor dem Zersplittern zu schützen.

Gegenstand der US 2017/119450 A1 ist ein Ballonkatheter, der in der Lage ist, die Temperatur eines Blutgefäßes zu erhöhen, und der unter anderem ein Führungsdrahtrohr, ein Innenrohr, ein Außenrohr, ein wärmeerzeugendes Element, ein Lichtleiterelement und ein auf dem wärmeerzeugenden Element in radialer Richtung überlagertes Abdeckrohr umfasst, auf das eine lichtreflektierende Metallschicht laminiert ist.

Ferner ist ein piezoresistiver Tastsensor auf Basis einer Polymerfolie mit Mikrorissen bekannt, wobei die Risse durch die Selbstspannung einer auf die Polymerfolie (PDMS-Folie) aufgesputterten Metallschicht gebildet werden und als Sensormaterial Ti₃C₂ in die Risse des Tastsensors eingebettet ist (CHEN LONGLONG ET AL: "High-Tactile Sensitivity of Piezoresistive Sensors With a Micro-Crack Structure Induced by Thin Film Tension", IEEE ELECTRON DEVICE LETTERS, IEEE, USA, Bd. 40, Nr. 9, 1. September 2019 (2019-09-01), Seiten 1519-1521).

### AUFGABE UND LÖSUNG

Aufgabe der Erfindung ist es, ein Medizinprodukt und ein Verfahren zum Herstellen eines Medizinprodukts bereitzustellen, welche insbesondere die einleitend erwähnte Marktlücke adressieren.

Diese Aufgabe wird gelöst durch ein Medizinprodukt mit den Merkmalen gemäß unabhängigem Anspruch 1 sowie durch ein Verfahren zum Herstellen eines Medizinprodukts gemäß Anspruch 12. Bevorzugte Ausgestaltungen der Erfindung sind Gegenstand der abhängigen Ansprüche sowie der nachfolgenden Beschreibung. Der Wortlaut sämtlicher Ansprüche wird hiermit durch ausdrückliche Bezugnahme zum Inhalt der Beschreibung gemacht.

Gemäß einem ersten Aspekt betrifft die Erfindung ein Medizinprodukt in Form eines Ballonkatheters.

Das Medizinprodukt weist wenigstens eine Mehrschichtstruktur auf, die nachfolgende übereinanderliegend angeordnete Schichten aufweist oder aus nachfolgenden übereinanderliegend angeordneten Schichten besteht:
- wenigstens eine Metallschicht und
- wenigstens eine Elastomermaterialschicht.

Der Ausdruck "wenigstens eine Mehrschichtstruktur" kann im Sinne der vorliegenden Erfindung nur eine Mehrschichtstruktur oder eine Vielzahl von Mehrschichtstrukturen, d.h. zwei oder mehr Mehrschichtstrukturen, bedeuten.

Die Mehrschichtstruktur kann im Sinne der vorliegenden Erfindung insbesondere als laminat- und/oder membranförmige Struktur, insbesondere Metall-Polymer-Laminat-Komposit, vorzugsweise Metall-Polymer-Laminat-Membran, gestaltet sein. Ferner kann die Mehrschichtstruktur im Sinne der vorliegenden Erfindung insbesondere als Metall-Polymer-Mehrschicht-Komposit-Struktur, insbesondere Metall-Polymer-Zweischicht-Komposit-Struktur, bezeichnet werden.

Der Ausdruck "wenigstens eine Metallschicht" kann im Sinne der vorliegenden Erfindung nur eine Metallschicht oder eine Vielzahl von Metallschichten, d.h. zwei oder mehr Metallschichten, bedeuten.

Der Ausdruck "Metallschicht" bedeutet im Sinne der vorliegenden Erfindung eine Schicht, die wenigstens ein Metall, insbesondere in elementarer Form oder in Form einer Legierung, aufweist oder aus wenigstens einem Metall, insbesondere in elementarer Form oder in Form einer Legierung, besteht.

Der Ausdruck "wenigstens eine Elastomermaterialschicht" kann im Sinne der vorliegenden Erfindung nur eine Elastomermaterialschicht oder eine Vielzahl von Elastomermaterialschichten, d.h. zwei oder mehr Elastomermaterialschichten, bedeuten.

Der Ausdruck "Elastomermaterialschicht" bedeutet im Sinne der vorliegenden Erfindung eine Schicht, die wenigstens ein Elastomermaterial, insbesondere wenigstens ein Elastomer, vorzugsweise wenigstens ein hyperelastisches Polymer, aufweist oder aus wenigstens einem Elastomermaterial, insbesondere wenigstens einem Elastomer, vorzugsweise wenigstens einem hyperelastischen Polymer, besteht.

Unter dem Ausdruck "hyperelastisches Polymer" soll im Sinne der vorliegenden Erfindung ein Polymer, insbesondere ein Elastomer mit hyperelastischen Eigenschaften, insbesondere hoher reversibler Dehnbarkeit, verstanden werden.

Die wenigstens eine Metallschicht weist vorzugsweise eine variierende Oberflächenmorphologie auf. Die variierende Oberflächenmorphologie ist vorzugsweise dazu ausgebildet, die Sensitivität, insbesondere das resistive Verhalten, gegenüber mechanischer Belastung, insbesondere Dehnung, zu beeinflussen, insbesondere zu erhöhen oder zu erniedrigen.

Die Erfindung beruht insbesondere auf der überraschenden Erkenntnis, dass die erfindungsgemäß vorgesehene Mehrschichtstruktur als Sensoreinheit in der Medizin, insbesondere zur Ermittlung der Gewebeelastizität, vorzugsweise der Elastizität von Hohlorganen, wie insbesondere Blutgefäßen, verwendbar ist.

In Ausgestaltung der Erfindung weist die wenigstens eine Elastomermaterialschicht wenigstens ein Elastomermaterial, insbesondere hyperelastisches Material, auf oder besteht aus einem Elastomermaterial, insbesondere hyperelastischen Material, das ausgewählt ist aus der Gruppe bestehend aus Elastomere, thermoplastische Elastomere, thermoplastische Polyamidelastomere, thermoplastische Copolyesterelastomere, thermoplastische Elastomere auf Olefinbasis, thermoplastische Styrol-Blockcopolymere, thermoplastische Elastomere auf Urethanbasis, thermoplastische Vulkanisate auf Olefinbasis, vernetzte thermoplastische Elastomere auf Olefinbasis, Vulkanisate von Naturkautschuken, Vulkanisate von Synthesekautschuken, Styrol-Butadien-Kautschuk, Butadien-Kautschuk (BR), AcrylnitrilButadien-Kautschuk (NBR), Butylkautschuk (IIR), Ethylen-Propylen-Dien-Kautschuk (EPDM), Chloropren-Kautschuk (CR), Polyisoprenkautschuk (IR), Polyalkylsiloxane, Polydimethylsiloxan, Silikonkautschuke, Silikonelastomere, Methyl-Silikon, Vinyl-Methyl-Silikon, Phenyl-Vinyl-Methyl-Silikon, Phenyl-modifiziertes Silikon, Fluoroalkyl-Silikon, Fluor-Vinyl-Methyl-Silikon und Mischungen von wenigstens zwei der vorgenannten Elastomermaterialien.

Vorzugsweise weist die wenigstes eine Elastomermaterialschicht ein Polyalkylsiloxan, insbesondere Polydimethylsiloxan, auf oder besteht aus Polyalkylsiloxan, insbesondere Polydimethylsiloxan.

Weiter kann die wenigstens eine Elastomermaterialschicht insbesondere folienförmig gestaltet sein.

Insbesondere kann die wenigstens eine Elastomermaterialschicht, insbesondere unmittelbar oder nicht unmittelbar, auf einer Oberfläche, insbesondere Außen- und/oder Innenoberfläche, vorzugsweise Außenoberfläche, des Medizinprodukts ausgebildet sein.

In weiterer Ausgestaltung der Erfindung weist die wenigstens eine Elastomermaterialschicht eine Schichtdicke von 0,0001 mm bis 0,2 mm, insbesondere 0,0005 mm bis 0,1 mm, vorzugsweise 0,001 mm bis 0,05 mm, auf.

In weiterer Ausgestaltung der Erfindung weist die wenigstens eine Metallschicht wenigstens ein Metall, insbesondere in elementarer Form oder in Form einer Legierung, auf oder besteht aus wenigstens einem Metall, insbesondere in elementarer Form oder in Form einer Legierung, das ausgewählt ist aus der Gruppe bestehend aus Gold, Platin, Indium, Zinn, Kupfer, Silber, Gallium und Mischungen, insbesondere Legierungen, von wenigstens zwei der vorgenannten Metalle.

Weiter kann die wenigstens eine Metallschicht insbesondere folienförmig gestaltet sein.

Weiter kann die wenigstens eine Metallschicht wenigstens abschnitts- oder bereichsweise, insbesondere nur abschnitts- oder bereichsweise oder durchgehend, serpentinenförmig gestaltet sein bzw. die wenigstens eine Elastomermaterialschicht bedecken oder beschichten.

Bevorzugt ist die Schichtdicke der wenigstens einen Metallschicht geringer als die Schichtdicke der wenigstens einen Elastomermaterialschicht.

In weiterer Ausgestaltung der Erfindung weist die wenigstens eine Metallschicht eine Schichtdicke von ≤ 150 nm, insbesondere 10 nm bis 100 nm, vorzugsweise 40 nm bis 80 nm, auf.

In weiterer Ausgestaltung der Erfindung ist die wenigstens eine Elastomermaterialschicht von der wenigstens einen Metallschicht, insbesondere unmittelbar oder nicht unmittelbar, bedeckt oder beschichtet. Weiter kann die wenigstens eine Elastomermaterialschicht von der wenigstens einen Metallschicht grundsätzlich vollständig, d.h. durchgehend oder vollflächig, oder nur teilweise, d.h. abschnitts- oder bereichsweise, bedeckt oder beschichtet sein. Vorzugsweise ist die wenigstens eine Elastomermaterialschicht von der wenigstens einen Metallschicht jedoch nur teilweise bedeckt oder beschichtet. Besonders bevorzugt ist die wenigstens eine Elastomermaterialschicht von der wenigstens einen Metallschicht unmittelbar und nur abschnitts- oder bereichsweise bedeckt oder beschichtet.

In weiterer Ausgestaltung der Erfindung ist zwischen der wenigstens einen Metallschicht und der wenigstens einen Elastomermaterialschicht wenigstens eine Adhäsionsschicht angeordnet oder ausgebildet.

Der Ausdruck "wenigstens eine Adhäsionsschicht" kann im Sinne der vorliegenden Erfindung nur eine Adhäsionsschicht oder eine Vielzahl von Adhäsionsschichten, d.h. zwei oder mehr Adhäsionsschichten, bedeuten.

Der Ausdruck "Adhäsionsschicht" bedeutet im Sinne der vorliegenden Erfindung eine Schicht, die wenigstens ein Material aufweist oder aus wenigstens einem Material besteht, das in der Lage ist, eine Anhaftung der wenigstens einen Metallschicht auf der wenigstens einen Elastomermaterialschicht zu ermöglichen und/oder zu verbessern.

Weiter kann die wenigstens eine Adhäsionsschicht die wenigstens eine Elastomermaterialschicht unmittelbar oder nicht unmittelbar bedecken oder beschichten. Insbesondere kann die wenigstens eine Adhäsionsschicht die wenigstens eine Elastomermaterialschicht vollständig, d.h. durchgehend oder vollflächig, oder nur teilweise, d.h. abschnitts- oder bereichsweise, bedecken oder beschichten. Vorzugsweise bedeckt die wenigstens eine Adhäsionsschicht die wenigstens eine Elastomermaterialschicht jedoch nur teilweise.

Weiter kann die wenigstens eine Metallschicht die wenigstens eine Adhäsionsschicht unmittelbar oder nicht unmittelbar bedecken oder beschichten. Insbesondere kann die wenigstens eine Metallschicht die wenigstens eine Adhäsionsschicht vollständig, d.h. durchgehend oder vollflächig, oder nur teilweise, d.h. abschnitts- oder bereichsweise, bedecken oder beschichten. Vorzugsweise bedeckt die wenigstens eine Metallschicht die wenigstens eine Adhäsionsschicht jedoch vollständig.

In weiterer Ausgestaltung der Erfindung ist die wenigstens eine Adhäsionsschicht, insbesondere unmittelbar und vorzugswiese vollständig, von der wenigstens einen Metallschicht und die wenigstens eine Elastomermaterialschicht, insbesondere unmittelbar und vorzugsweise nur abschnitts- oder bereichsweise, von der wenigstens einen Adhäsionsschicht bedeckt oder beschichtet.

In weiterer Ausgestaltung der Erfindung weist die wenigstens eine Adhäsionsschicht wenigstens ein Material auf oder besteht aus wenigstens einem Material, das ausgewählt ist aus der Gruppe bestehend aus Titan, Aluminium, Chrom und Mischungen von wenigstens zwei der vorgenannten Materialien.

Weiter kann die wenigstens eine Adhäsionsschicht insbesondere folienförmig gestaltet sein.

Weiter kann die wenigstens eine Adhäsionsschicht wenigstens abschnitts- oder bereichsweise, insbesondere nur abschnitts- oder bereichsweise oder durchgehend, serpentinenförmig gestaltet sein bzw. die wenigstens eine Elastomermaterialschicht bedecken oder beschichten.

Bevorzugt ist die Schichtdicke der wenigstens einen Adhäsionsschicht geringer als die Schichtdicke der wenigstens einen Elastomermaterialschicht.

Insbesondere kann die Schichtdicke der wenigstens einen Adhäsionsschicht geringer sein als die Schichtdicke der wenigstens einen Metallschicht.

Vorzugsweise weist die wenigstens eine Adhäsionsschicht eine Schichtdicke ≤ 20 nm, insbesondere 3 nm bis 20 nm, vorzugsweise 4 nm bis 10 nm, auf.

In weiterer Ausgestaltung der Erfindung sind/ist die wenigstens eine Metallschicht und/oder die wenigstens eine Adhäsionsschicht als Leiterbahn/Leiterbahnen ausgebildet. Die Leiterbahn/Leiterbahnen sind zum Ableiten eines elektrischen Messsignals eingerichtet. Zur Auswertung des elektrischen Messsignals können/kann die wenigstens eine Metallschicht und/oder die wenigstens eine Adhäsionsschicht bei dieser Ausführungsform der Erfindung elektrisch leitfähig mit einer Auswerteeinheit zur Auswertung des elektrischen Messsignals kontaktiert oder kontaktierbar sein.

In weiterer Ausgestaltung der Erfindung weist die wenigstens eine Mehrschichtstruktur ferner wenigstens eine zusätzliche Elastomermaterialschicht, d.h. nur eine zusätzliche Elastomermaterialschicht oder eine Vielzahl von, d.h. zwei oder mehr, Elastomermaterialschichten, auf. Bevorzugt ist die wenigstens eine Metallschicht, insbesondere unmittelbar oder nicht unmittelbar, vorzugsweise unmittelbar, von der wenigstens einen zusätzlichen Elastomermaterialschicht bedeckt oder beschichtet.

Bevorzugt ist die Schichtdicke der wenigstens einen Metallschicht geringer als die Schichtdicke der wenigstens einen zusätzlichen Elastomermaterialschicht.

Weiter bevorzugt ist die Schichtdicke der wenigstens einen Adhäsionsschicht geringer als die Schichtdicke der wenigstens einen zusätzlichen Elastomermaterialschicht.

Die wenigstens eine zusätzliche Elastomermaterialschicht kann eine Schichtdicke von 0,001 mm bis 0,15 mm, insbesondere 0,001 mm bis 0,1 mm, vorzugsweise 0,001 mm bis 0,05 mm, aufweisen.

Weiter kann die wenigstens eine zusätzliche Elastomermaterialschicht insbesondere folienförmig gestaltet sein.

Weiter können die wenigstens eine zusätzliche Elastomermaterialschicht und die wenigstens eine Elastomermaterialschicht gleich oder unterschiedlich gestaltet sein, insbesondere in Bezug auf das Elastomermtaterial und/oder die Schichtdicke.

Weiter kann die wenigstens eine zusätzliche Elastomermaterialschicht, insbesondere unabhängig von der wenigstens einen Elastomermtaterialschicht, ein Elastomermaterial, insbesondere hyperelastisches Material, aufweisen oder aus einem Elastomermaterial, insbesondere hyperelastischen Material, bestehen, das ausgewählt ist aus der Gruppe bestehend aus Elastomere, thermoplastische Elastomere, thermoplastische Polyamidelastomere, thermoplastische Copolyesterelastomere, thermoplastische Elastomere auf Olefinbasis, thermoplastische Styrol-Blockcopolymere, thermoplastische Elastomere auf Urethanbasis, thermoplastische Vulkanisate auf Olefinbasis, vernetzte thermoplastische Elastomere auf Olefinbasis, Vulkanisate von Naturkautschuken, Vulkanisate von Synthesekautschuken, Styrol-Butadien-Kautschuk, Butadien-Kautschuk (BR), AcrylnitrilButadien-Kautschuk (NBR), Butylkautschuk (IIR), Ethylen-Propylen-Dien-Kautschuk (EPDM), Chloropren-Kautschuk (CR), Polyisoprenkautschuk (IR), Polyalkylsiloxane, Polydimethylsiloxan, Silikonkautschuke, Silikonelastomere, Methyl-Silikon, Vinyl-Methyl-Silikon, Phenyl-Vinyl-Methyl-Silikon, Phenyl-modifiziertes Silikon, Fluoroalkyl-Silikon, Fluor-Vinyl-Methyl-Silikon und Mischungen von wenigstens zwei der vorgenannten Elastomermaterialien.

Vorzugsweise weist die wenigstes eine zusätzliche Elastomermaterialschicht ein Polyalkylsiloxan, insbesondere Polydimethylsiloxan, auf oder besteht aus Polyalkylsiloxan, insbesondere Polydimethylsiloxan.

In weiterer Ausgestaltung der Erfindung weist die wenigstens eine Mehrschichtstruktur oder die wenigstens eine Metallschicht eine variierende Oberflächenmorphologie auf. Insbesondere kann die wenigstens eine Mehrschichtstruktur oder die wenigstens eine Metallschicht wellige Oberflächenbereiche und/oder nicht wellige Oberflächenbereiche aufweisen. Vorzugsweise weist die wenigstens eine Mehrschichtstruktur oder die wenigstens eine Metallschicht Oberflächenbereiche, insbesondere wellige Oberflächenbereiche und/oder nicht wellige Oberflächenbereiche, mit Rissen, insbesondere Mikrorissen, und/oder Oberflächenbereiche, insbesondere wellige Oberflächenbereiche und/oder nicht wellige Oberflächenbereiche, ohne Risse auf. Besonders bevorzugt weist die wenigstens eine Mehrschichtstruktur oder die wenigstens eine Metallschicht, insbesondere wellige und/oder nicht wellige, Oberflächenbereiche mit Rissen, insbesondere Mikrorissen, und wellige Oberflächenbereiche ohne Risse auf. Durch eine variierende Oberflächenmorphologie kann vorteilhafterweise die Sensitivität der wenigstens einen Metallschicht selektiv gegenüber mechanischer Belastung, insbesondere Dehnung, beeinflusst, insbesondere erhöht oder verringert, werden.

Unter dem Ausdruck "Mikrorisse" sollen im Sinne der vorliegenden Erfindung Risse verstanden werden, die wenigstens eine Abmessung, insbesondere ausgewählt aus der Gruppe bestehend aus Länge, Breite, Tiefe und Kombinationen von wenigstens zwei der vorgenannten Abmessungen, im Mikrometer- und/oder Nanometerbereich, insbesondere in einem Bereich von 1 nm bis 5 µm, insbesondere 10 nm bis 2 µm, vorzugsweise 20 nm bis 1 µm, aufweisen. Die Ausbildung der Risse ist vorzugsweise abhängig von angewandten Beschichtungsparametern und/oder einer Vorbehandlung der Oberfläche der wenigstens einen Elastomermaterialschicht.

Unter dem Ausdruck "wellige Oberflächenbereiche ohne Risse" sollen im Sinne der vorliegenden Erfindung, vorzugsweise durch Druckspannung innerhalb der wenigstens einen Metallschicht entstehende, sich periodisch wiederholende wellenartige Verformungen der Oberfläche der wenigstens einen Metallschicht, insbesondere durch Verformung der darunter liegenden wenigstens einen Elastomermaterialschicht, verstanden werden. Bevorzugt weisen die welligen Oberflächenbereiche ohne Risse wenigstens eine Abmessung, insbesondere ausgewählt aus der Gruppe bestehend aus Periodenlänge und Amplitude und Kombinationen der zwei vorgenannten Abmessungen, im Mikrometer- und/oder Nanometerbereich, insbesondere in einem Bereich von 10 nm bis 15 µm, insbesondere 20 nm bis 10 µm, vorzugsweise 40 nm bis 6 µm, auf.

In weiterer Ausgestaltung der Erfindung ist die wenigstens eine Mehrschichtstruktur Teil einer Oberfläche, insbesondere einer Außen- und/oder Innenoberfläche, des Medizinprodukts. Insbesondere kann das Medizinprodukt, insbesondere eine Außen- und/oder Innenoberfläche des Medizinprodukts, vorzugsweise wenigstens abschnitts- oder bereichsweise, mit der wenigstens einen Mehrschichtstruktur beschichtet sein.

In weiterer Ausgestaltung der Erfindung ist die wenigstens eine Mehrschichtstruktur Teil einer Wandung des Medizinprodukts oder in einer Wandung des Medizinprodukts integriert oder innerhalb einer Wandung des Medizinprodukts ausgebildet.

Weiter kann die wenigstens eine Mehrschichtstruktur grundsätzlich in Längs- und/oder Quer- oder Umfangsrichtung des Medizinprodukts ausgebildet sein. Bevorzugt ist die wenigstens eine Mehrschichtstruktur in Umfangsrichtung des Medizinprodukts ausgebildet.

Weiter kann die wenigstens eine Mehrschichtstruktur streifenförmig gestaltet sein.

Insbesondere kann das Medizinprodukt eine Vielzahl von insbesondere streifenförmigen Mehrschichtstrukturen aufweisen, die in Längs- und/oder Quer- oder Umfangsrichtung des Medizinprodukts ausgebildet sind.

In weiterer Ausgestaltung der Erfindung ist das Medizinprodukt gewölbt, insbesondere zylindrisch gewölbt, gestaltet.

Vorzugsweise ist das Medizinprodukt zylindrisch, insbesondere hohlzylindrisch, oder schlauchförmig gestaltet.

Das Medizinprodukt ist als Ballonkatheter gestaltet.

Unter dem Ausdruck "Ballonkatheter" soll im Sinne der vorliegenden Erfindung ein Katheter, insbesondere aus Kunststoff, Gummi, Silikon, Metall oder Glas, verstanden werden, der mit einem Ballon versehen ist. Der Ballon kann entweder mit Druckluft oder Flüssigkeit inflatiert, d.h. aufgedehnt, werden.

Beispielsweise kann der Katheter als Angiographiekatheter, Embolektomiekatheter, Herzkatheter, peripherer Venenkatheter, zentraler Venenkatheter, Broviac-Katheter, Fogarty-Katheter, Hickman-Katheter, Swan-Ganz-Katheter, Endotrachealkatheter, Bronchialkatheter, Blasenkatheter, Nephrostomie-Katheter, Ureterkatheter, Enterostomiekatheter, Shaldon-Katheter, Demers-Katheter, Ballonspülkatheter, Einschwemmkatheter, Pigtail-Katheter oder Doppel-J-Katheter gestaltet sein.

Besonders bevorzugt ist der Katheter als Ballonkatheter, insbesondere als Kunststoffkatheter, der an seiner Spitze einen mit Druckluft oder Flüssigkeit entfaltbaren Ballon (Okklusionsballon) trägt, gestaltet. Der Ballonkatheter kann beispielsweise für die Angioplastie, insbesondere perkutane transluminale Koronarangioplastie (PTCA), für die Embolektomie, Valvuloplastie, insbesondere bei stenosierten Herzklappen, Katheterisierung der Harnblase oder für die Blockade oder Dilatation der Bronchien gestaltet sein.

Das Medizinprodukt ist als Ballonkatheter gestaltet, wobei der Ballon des Ballonkatheters die wenigstens eine Mehrschichtstruktur aufweist.

Die wenigstens eine Mehrschichtstruktur definiert wenigstens eine Sensoreinheit, insbesondere wenigstens eine taktile Sensoreinheit. Bevorzugt ist die wenigstens eine Sensoreinheit in Form wenigstens eines, insbesondere schichtweise aufgebauten, Sensorstacks, ausgebildet. Die wenigstens eine Sensoreinheit ist vorzugsweise zum Erfassen einer Messgröße, insbesondere physikalischen Messgröße, und einer Wandlung derselben in ein Messsignal, vorzugsweise elektrisches Messsignal, eingerichtet. Bei der Messgröße kann es sich insbesondere um eine mechanische Eigenschaft, wie beispielsweise Dehnbarkeit oder Elastizität, oder ein Struktur- und/oder Funktionsparameter eines Gewebes, insbesondere eines Hohlorgans, vorzugsweise eines Blutgefäßes, insbesondere einer Arterie, handeln. Alternativ oder in Kombination kann es sich bei der Messgröße um eine Temperatur, einen hydrostatischen Druck oder eine sonstige Messgröße, insbesondere von therapeutischem und/oder diagnostischem Interesse, handeln.

Bevorzugt ist die wenigstens eine Sensoreinheit zum Erfassen einer Messgröße, wie beispielsweise Dehnbarkeit oder Elastizität, die ein Blutgefäß, insbesondere eine Arterie, charakterisiert, ausgebildet. Dadurch kann vorteilhafterweise eine Diagnostik, insbesondere intraoperative Diagnostik, und/oder Therapie von Blutgefäßerkrankungen, insbesondere arteriosklerotisch bedingten Erkrankungen, optimiert werden.

Ist das Medizinprodukt als Ballonkather ausgebildet und bildet die wenigstens eine Mehrschichtstruktur ein Teil des Ballons des Ballonkatheters, kann beispielsweise vorteilhafterweise die Elastizität eines Blutgefäßes, insbesondere einer Arterie, ermittelt, insbesondere taktil ermittelt, werden, indem über die Sensoreinheit ein Widerstand, insbesondere elektrischer Widerstand, erfasst wird, der mit einer Expansion bzw. Inflation des Ballons des Ballonkatheters bzw. mit dem Druck, der zum Expandieren bzw. Inflatieren des Ballons des Ballonkatheters verwendet wird, korreliert, insbesondere linear und/oder nichtlinear sowie reversibel zunimmt, und insbesondere durch Vergleich mit einem Referenzverhalten ohne Kontakt mit umgebendem Gewebe interpretiert werden kann.

Gemäß einem zweiten Aspekt betrifft die Erfindung ein Verfahren zum Herstellen eines Medizinprodukts gemäß erstem Erfindungsaspekt. Das Produkt kann insbesondere in Form eines Metall-Polymer-Laminat-Komposits, insbesondere einer Metall-Polymer-Laminat-Membran, insbesondere dünnen Metall-Polymer-Laminat-Membran, gestaltet sein. Das Verfahren weist die nachfolgenden Schritte auf:
a) Herstellen wenigstens einer Mehrschichtstruktur mit wenigstens einer Metallschicht, insbesondere mit variierender Oberflächenmorphologie, und wenigstens einer Elastomermaterialschicht und
b) Transferieren, d.h. Übertragen, der wenigstens einen Mehrschichtstruktur auf ein Substrat unter Ausbildung eines mit der wenigstens einen Mehrschichtstruktur versehenen Substrats.

Vorzugsweise wird der Schritt b) derart ausgeführt, dass keine relevanten Veränderungen der Oberflächenmorphologie der wenigstens einen Metallschicht, insbesondere vor Einsatz des Substrats als Medizinprodukt, stattfinden.

Als Substrat kann grundsätzlich ein planares, d.h. ebenes oder flaches, Substrat oder, was bevorzugt ist, ein nicht planares, insbesondere gewölbtes, Substrat verwendet werden. Bevorzugt wird ein zylindrisches oder zylindrisch gewölbtes, insbesondere hohlzylindrisches oder schlauchförmiges, oder konisch gestaltetes Substrat verwendet. Vorzugsweise handelt es sich bei dem Substrat um eine Vorstufe, insbesondere einen Rohling, eine Komponente wie ein Bauteil oder ein Halbfabrikat, eines Medizinprodukts.

Bevorzugt wird die wenigstens eine Mehrschichtstruktur beim Durchführen von Schritt a) auf einer Transferfolie, insbesondere semikomplianten Transferfolie, hergestellt, wobei die Transferfolie vorzugsweise auf einem Träger aufgebracht ist. Mit anderen Worten wird die wenigstens eine Mehrschichtstruktur vorzugsweise auf einem mit einer Transferfolie funktionalisierten oder beschichteten Träger hergestellt. Bevorzugt ist die Transferfolie von dem Träger entfernbar, insbesondere ablösbar. Als Träger kann beispielsweise ein Wafer, insbesondere in Form einer kreisförmigen oder viereckigen, insbesondere rechteckigen oder quadratischen, Scheibe verwendet werden. Ein geeigneter Wafer kann aus einem ein- oder polykristallinen Rohling (sogenannter Ingot) hergestellt werden. Weiter kann der Wafer monokristallines Silizium und/oder ein anderes Material, beispielsweise ausgewählt aus der Gruppe bestehend aus Siliziumcarbid, Galliumarsenid, Indiumphosphid und Mischungen von wenigstens zwei der vorgenannten Materialien, aufweisen oder aus einem solchen Material/solchen Materialien bestehen. Weiter kann der Wafer beispielsweise eine Dicke von 0,5 mm besitzen. Als Transferfolie kann beispielsweise eine Folie verwendet werden, die eine klebende Schicht oder Seite, beispielsweise auf Acryl- und/oder Methacrylbasis, aufweist. Vorzugsweise wird durch eine Exposition mit UV-Licht eine Reduktion der Klebefähigkeit der klebenden Schicht oder Seite herbeigeführt, wodurch die Trägerfolie vorteilhafterweise leichter von dem Träger abgelöst werden kann, allerdings die Adhäsion gegenüber dem Träger stark genug bleibt, um ein vorzeitiges Ablösen der Trägerfolie während weiterer Prozessschritte zu vermeiden., Neben der klebenden Schicht kann die Transferfolie eine weitere Schicht, insbesondere eine nichtklebende Schicht, beispielsweise aus Polyethylenterephthalat, aufweisen. Die Transferfolie kann beispielsweise mit einer Schichtdicke von 40 µm bis 240 µm, insbesondere 60 µm bis 155 µm, vorzugsweise 75 µm bis 120 µm, auf das Substrat aufgebracht sein. Vorzugsweise ist das Substrat einseitig mit der Transferfolie beschichtet, insbesondere beklebt.

Vorzugsweise wird die wenigstens eine Elastomermaterialschicht beim Durchführen von Schritt a), insbesondere mittels Rotationsbeschichtung, auf die Transferfolie aufgebracht. Grundsätzlich kann die wenigstens eine Elastomermaterialschicht unmittelbar oder nicht unmittelbar auf die Transferfolie aufgebracht werden, vorzugsweise jedoch nach einer UV-Exposition der Transferfolie zur Adhäsionsreduktion, um einen Kontakt der wenigstens einen Elastomermaterialschicht mit UV-Licht und eine damit verbundene mögliche Degradierung der wenigstens einen Elastomermaterialschicht, zu vermeiden. Bevorzugt wird die wenigstens eine Elastomermaterialschicht unmittelbar auf die Transferfolie aufgebracht. Weiter kann die wenigstens eine Elastomermaterialschicht nur teilweise, d.h. nur abschnitts- oder bereichsweise, oder durchgehend, d.h. vollflächig, auf die Transferfolie aufgebracht werden. Bevorzugt wird die wenigstens eine Elastomermaterialschicht durchgehend auf die Transferfolie aufgebracht. Vorzugsweise wird anschließend die wenigstens eine Metallschicht auf die wenigstens eine Elastomermaterialschicht aufgebracht. Grundsätzlich kann die wenigstens eine Metallschicht unmittelbar oder nicht unmittelbar auf die wenigstens eine Elastomermaterialschicht aufgebracht werden. Weiter kann die wenigstens eine Metallschicht nur teilweise, d.h. nur abschnitts- oder bereichsweise, oder durchgehend, d.h. vollflächig, auf die wenigstens eine Elastomermaterialschicht aufgebracht werden. Bevorzugt wird die wenigstens eine Metallschicht durchgehend auf die wenigstens eine Elastomermaterialschicht aufgebracht. Beim Durchführen der Rotationsbeschichtung kann die Elastomermaterialschicht oder, genauer gesagt, das Elastomermaterial oder eine Vorstufe davon, insbesondere in Form eines härtbaren Prepolymers (vorzugsweise in Form eines viskosen, härtbaren Prepolymers ohne Lufteinschlüsse), beispielsweise mit einer Geschwindigkeit von 400 UpM (gesprochen: Umdrehungen pro Minute) bis 3000 UpM, insbesondere 500 UpM bis 2000 UpM, vorzugsweise 600 UpM bis 900 UpM, auf die Transferfolie aufgeschleudert werden. Weiter kann die Rotationsbeschichtung während eines Zeitraumes von 30 s bis 120 s, insbesondere 45 s bis 90 s, vorzugsweise 60 s bis 80 s, durchgeführt werden. Die Rotationsbeschichtung kann grundsätzlich mit variierenden Geschwindigkeiten und Zeitdauern durchgeführt werden. Dadurch kann vorteilhafterweise die Gleichmäßigkeit der resultierenden wenigstens einen Elastomermaterialschicht verbessert werden. Insbesondere bei hohen Rotationsgeschwindigkeiten kann dabei auch die Beschleunigung (in UpM/s, gesprochen: Umdrehungen pro Minute pro Sekunde) bis zu einer angestrebten Rotationsgeschwindigkeit variiert werden. Dadurch kann ein gleichmäßiges Fließverhalten und eine gleichmäßige Benetzung nach initialem Aufbringen der Vorstufe des Elastomermaterials, insbesondere des Prepoylmers, gewährleistet werden. Hierbei kann die Beschleunigung von 100 UpM/s bis 1000 UpM/s, insbesondere von 150 UpM/s bis 800 UpM/s, vorzugsweise von 200 UpM/s bis 400 UpM/s, variiert werden. Danach wird vorzugsweise ein Vakuum oder Unterdruck angelegt, beispielsweise für einen Zeitraum von 10 min bis 20 min. Hierdurch kann vorteilhafterweise die Ausbildung einer defektfreien und vorzugsweise geraden Elastomermaterialschicht, insbesondere in Form einer Membran, realisiert werden. Anschließend kann die Elastomermaterialschicht ausgehärtet werden, beispielsweise bei einer Temperatur von 25 °C bis 120 °C und insbesondere während eines Zeitraums von 30 min bis 48 h. Das Aushärten der Elastomermaterialschicht kann bevorzugt in einem Ofen durchgeführt werden.

Bevorzugt wird die wenigstens eine Metallschicht mithilfe eines die Oberfläche der wenigstens einen Elastomermaterialschicht strukturierenden Verfahrens, insbesondere mithilfe von Fotolithografie, und eines anschließend durchgeführten vakuumbasierten Beschichtungsverfahrens, insbesondere mithilfe von physikalischer Gasphasenabscheidung, vorzugsweise mittels thermischem Verdampfen, auf die wenigstens eine Elastomermaterialschicht aufgebracht, insbesondere unmittelbar oder nicht unmittelbar.

Vorzugsweise wird zuerst wenigstens eine Adhäsionsschicht mithilfe eines die Oberfläche der wenigstens einen Elastomermaterialschicht strukturierenden Verfahrens, insbesondere mithilfe von Fotolithografie, und eines anschließend durchgeführten vakuumbasierten Beschichtungsverfahrens, insbesondere mithilfe von physikalischer Gasphasenabscheidung, vorzugsweise mittels thermischem Verdampfen, wenigstens abschnitts- oder bereichsweise, insbesondere nur abschnitts- oder bereichsweise oder durchgehend, d.h. vollflächig, auf die wenigstens eine Elastomermaterialschicht aufgebracht, insbesondere unmittelbar oder nicht unmittelbar, und anschließend die wenigstens eine Metallschicht mithilfe eines die Oberfläche der wenigstens einen Elastomermaterialschicht strukturierenden Verfahrens, insbesondere mithilfe von Fotolithografie, und eines anschließend durchgeführten vakuumbasierten Beschichtungsverfahrens, insbesondere mithilfe von physikalischer Gasphasenabscheidung, auf die wenigstens eine Adhäsionsschicht aufgebracht, insbesondere unmittelbar oder nicht unmittelbar, vorzugsweise unmittelbar und unter Beibehaltung des Vakuums oder des Unterdrucks, insbesondere bei Verwendung von reaktiven Metallen zur Ausbildung der wenigstens einen Adhäsionsschicht.

Zum Durchführen der Fotolithografie wird vorzugsweise zunächst ein Fotolack auf die wenigstens eine Elastomermaterialschicht aufgebracht. Als Fotolack kann grundsätzlich ein Positivlack oder ein Negativlack verwendet werden. Unter einem Positivlack versteht man einen Fotolack, der in den veränderten, insbesondere belichteten, Bereichen entfernt wird. Unter einem Negativlack versteht man einen Fotolack, der in den unveränderten, insbesondere unbelichteten, Bereichen entfernt wird. Vorzugsweise wird als Fotolack ein Negativlack verwendet. Bevorzugt wird der Fotolack mittels Rotationsbeschichtung auf die wenigstens eine Elastomermaterialschicht aufgebracht. Die Rotationsbeschichtung kann beispielsweise mit einer Geschwindigkeit von 1000 UpM bis 4000 UpM, insbesondere 2000 UpM bis 3000 UpM, und insbesondere während eines Zeitraums von 45 s bis 90 s, bevorzugt 60 s bis 75 s, durchgeführt werden. Anschließend kann der auf die wenigstens eine Elastomermaterialschicht aufgebrachte Fotolack erhitzt werden, insbesondere bei einer Temperatur von 90 °C bis 110 °C und insbesondere während eines Zeitraums von 90 s bis 2 min. Dadurch können im Fotolack enthaltene Lösungsmittel, insbesondere Propylenglykolmonomethyletheracetat (PGMEA), desorbieren, wodurch der Fotolack stabilisiert wird. Anschließend wird eine Fotomaske auf den auf die wenigstens eine Elastomermaterialschicht aufgebrachten Fotolack aufgelegt. Als Fotomaske kann grundsätzlich eine aus, insbesondere hochreinem, Quarzglas oder Calciumfluorid bestehende Maske verwendet werden. Die Fotomaske kann zudem beispielsweise auf einer Seite mit einer Chromschicht, insbesondere strukturierten Chromschicht, versehen sein.

Als nächster Schritt wird beim Durchführen der Fotolithografie bevorzugt eine Belichtung durchgeführt. Dadurch wird vorteilhafterweise das Bild der Fotomaske auf den Fotolack übertragen. So entsteht eine lithografische Maske, die insbesondere eine weitere Bearbeitung, bevorzugt durch chemische und/oder physikalische Prozesse, ermöglicht. Die Belichtung kann mit einer Strahlungsdosis von 50 mJ/cm² bis 150 mJ/cm², insbesondere 60 mJ/cm² bis 120 mJ/cm², vorzugsweise 70 mJ/cm² bis 100 mJ/cm², durchgeführt werden. Als Strahlungsquelle kann beispielsweise eine Quecksilberdampflampe, insbesondere Quecksilberdampf-Hochdrucklampe, verwendet werden. Unter einer Quecksilberdampf-Hochdrucklampe versteht man eine Quecksilberdampflampe, die einen Betriebsdruck bis etwa 1 MPa hat, den sie nach wenigen Minuten Erwärmung erreicht. Das zum Belichten des Fotolacks eingesetzte Licht kann insbesondere eine Wellenlänge von 280 nm bis 700 nm, insbesondere 300 nm bis 500 nm, vorzugsweise 350 nm bis 450 nm, aufweisen.

Nach dem Belichten kann ein Temperschritt, insbesondere bei einer Temperatur von 100 °C bis 180 °C, bevorzugt 100 °C bis 120 °C, und insbesondere während eines Zeitraums von 2 min 30 s bis 3 min 30 s, bevorzugt 2 min 50 s bis 3 min, durchgeführt werden.

Nach dem Temperschritt kann der belichtete Fotolack zusammen mit dem Träger, der Transferfolie und der wenigstens einen Elastomermaterialschicht direkt in einen vorgeheizten Ofen transferiert werden. Der Ofen ist vorzugsweise auf eine Temperatur vorgeheizt, die niedriger ist als die Temperatur des im vorherigen Absatz beschriebenen Temperschrittes. Bevorzugt ist der Ofen auf eine Temperatur von 100°C vorgeheizt..

Nach dem oben erwähnten Temperschritt und insbesondere vor dem nachfolgend beschriebenen Entwickeln des Fotolacks, vorzugsweise nach Abschalten des oben erwähnten Ofens, kann der Fotolack gekühlt werden, insbesondere auf eine Temperatur von 18 °C bis 30 °C, vorzugsweise 20 °C bis 25 °C. Weiter kann der Fotolack insbesondere über einen Zeitraum von 5 h gekühlt werden. Dadurch kann vorteilhafterweise eine Rissbildung innerhalb des Fotolacks verhindert werden.

Schließlich wird beim Durchführen der Fotolithografie vorzugsweise ein Entwickeln des Fotolacks durchgeführt. Grundsätzlich können dadurch die belichteten Stellen des Fotolacks oder, insbesondere wenn der Fotolack unter Licht aushärtet, die unbelichteten Stellen des Fotolacks aufgelöst werden. Bevorzugt wird zum Entwickeln des Fotolacks eine Entwicklerlösung verwendet. Dadurch können die löslichen Bereiche des Fotolacks gelöst, beispielsweise nasschemisch durch Sprühen, Tauchen oder Auftropfen, und anschließend entfernt werden. Als Entwicklerlösung kann beispielsweise eine wässrige Lösung verwendet werden, die Tetramethylammoniumhydroxid (TMAH) enthält. Alternativ kann eine Entwicklerlösung auf Basis von gepufferter Natronlauge oder gepufferter Kalilauge oder auf Basis von Natrium-Phosphat und Natrium-Metasilikat verwendet werden. Das Entwickeln des Fotolacks kann beispielsweise während eines Zeitraums von 1 min 30 s bis 2 min 20 s, insbesondere 1 min 50 s bis 2 min 10, vorgenommen werden. Anschließend können eine Spülung mit Wasser, insbesondere deionisiertem Wasser, und ein Trocknen unter Inertgas, insbesondere Stickstoff, erfolgen.

Alternativ kann zum Strukturieren der Oberfläche der wenigstens einen Elastomermaterialschicht eine Loch- oder Schablonenmaske, beispielsweise aufweisend oder bestehend aus Nickel oder einer Nickel-Phosphor-Legierung, verwendet und anschließend eine Belichtung mit UV-Strahlung durchgeführt werden.

Um eine variierende Oberflächenmorphologie nach Abscheidung der wenigstens einen Metallschicht mit oder ohne wenigstens einer Adhäsionsschicht in unterschiedlichen Bereichen beeinflussen zu können, kann, insbesondere vor dem Einbringen der Elastomermaterialschicht in eine Beschichtungskammer, eine selektive Plasmabehandlung der Elastomermaterialschicht-Oberfläche vorgenommen werden. Hierzu kann die für diesen Zweck vorgenommene fotolithografische Maskierung und/oder die korrespondierende Schablonenmaske so ausgelegt sein, dass nur Teile der später mit der wenigstens einen Metallschicht und optional der wenigstens einen Adhäsionsschicht zu beschichteten Flächen freiliegen. Durch Einbringen in eine Plasmakammer und durch Exposition der freiliegenden Flächen gegenüber einem Plasma, können die Oberflächeneigenschaften der Elastomermaterial-Oberfläche verändert und so die später während der Abscheidung entstehende Oberflächenmorphologie der mindestens einen Metallschicht in diesen Bereichen beeinflusst werden. Die Beeinflussung der Oberfläche der wenigstens einen Elastomermaterialschicht durch das Plasma hängt auch von der Wahl des Prozessgases sowie der Prozessparameter ab. Um das Schichtwachstum so zu beeinflussen, das eine wellenförmige geschlossene Oberfläche entsteht, kann insbesondere Sauerstoff als Prozessgas verwendet werden, vorzugsweise bei Plasmaleistungen von 50 W bis 150 W, einem Prozessdruck von 0,5 mbar bis 1,5 mbar und einem Prozessgasmengenstrom von 100 sccm (gesprochen: Standardkubikzentimeter pro Minute) bis 700 sccm, vorzugsweise von 300 sccm bis 500 sccm. Die Zeitdauer der Plasmabehandlung kann 5 s bis 40 s, insbesondere 10 s bis 35 s, vorzugsweise 15 s bis 30 s, betragen.

Die physikalische Gasphasenabscheidung kann ausgewählt sein aus der Gruppe bestehend aus thermisches Verdampfen, Elektronenstrahlverdampfen, Laserstrahlverdampfen, Lichtbogenverdampfen, Molekularstrahlepitaxie, Sputtern, Ionenstrahl-gestützte Deposition, Ionenplattieren, ICB-Technik und eine Kombination von wenigstens zwei der vorgenannten physikalischen Gasphasenabscheidungen. Generell wird bei der physikalischen Gasphasenabscheidung das abzuscheidende Material in fester Form in einer meist evakuierten Beschichtungskammer durchgeführt. Durch den Beschuss mit Laserstrahlen, magnetisch abgelenkten Ionen oder Elektronen, durch Lichtbogenentladung oder durch Erhitzen (insbesondere bis nahe an den Siedepunkt) wird das Material, das auch als Target bezeichnet werden kann, verdampft. Das verdampfte Material bewegt sich entweder ballistisch oder durch elektrische Felder geführt durch die Beschichtungskammer und trifft dabei auf die zu beschichtenden Teile, wo es zur Schichtbildung kommt. Damit die Dampfteilchen die zu beschichtenden Teile auch erreichen und nicht durch Streuung an Gasteilchen verloren gehen, wird in der Regel im Unterdruck gearbeitet. Typische Arbeitsdrücke liegen im Bereich von 10⁻⁴ Pa bis 10 Pa.

Vorzugsweise wird nach dem Aufbringen der wenigstens einen Metallschicht auf die wenigstens eine Adhäsionsschicht und/oder die wenigstens eine Elastomermaterialschicht die Fotomaske, bestehend aus den unbelichteten Bereichen des Fotolacks, entfernt. Bevorzugt werden die unbelichteten Bereiche des Fotolacks mit einem organischen Lösungsmittel, wie beispielsweise Aceton, entfernt.

Im Falle der Verwendung einer Loch- oder Schablonenmaske zum Strukturieren der Oberfläche der wenigstens einen Elastomermaterialschicht wird die Maske nach dem Aufbringen der wenigstens einen Metallschicht auf die wenigstens eine Adhäsionsschicht und/oder wenigstens eine Elastomermaterialschicht vorzugsweise entfernt, beispielsweise durch Abziehen.

Bevorzugt wird beim Durchführen von Schritt a) auf die wenigstens eine Metallschicht wenigstens eine zusätzliche Elastomermaterialschicht, insbesondere mittels Rotationsbeschichtung, aufgebracht. Bezüglich weiterer Merkmale und Vorteile, insbesondere in Bezug auf die wenigstens eine zusätzliche Elastomermaterialschicht sowie die Rotationsbeschichtung, wird vollständig auf die bisherige Beschreibung Bezug genommen. Die dort insbesondere in Bezug auf die wenigstens eine Elastomermaterialschicht beschriebenen Merkmale und Vorteile gelten sinngemäß auch für die wenigstens eine zusätzliche Elastomermaterialschicht.

Zur Kontaktierung der wenigstens einen Metallschicht mit der wenigstens einen Adhäsionsschicht zwischen der wenigstens einen Elastomermaterialschicht und der wenigstens einen zusätzlichen Elastomermaterialschicht können unterschiedliche Kontaktierungstechniken zur Anwendung kommen. Beispielsweise kann eine Kontaktierung in vordefinierten Taschen innerhalb der wenigstens einen zusätzlichen Elastomermaterialschicht erfolgen, in denen beispielsweise mit Hilfe von leitfähigen, viskosen Pasten eine elektrisch leitende Verbindung zwischen definierten Kontaktpads der wenigstens einen Metallschicht mit der wenigstens einen Adhäsionsschicht und Zuleitungen, die zu einer Auswerteperipherie führen und die in der wenigstens einen zusätzlichen Elastomermaterialschicht eingebettet sind, hergestellt wird.

Weiter bevorzugt wird die wenigstens eine Mehrschichtstruktur zusammen mit der Transferfolie vor dem Durchführen von Schritt b) von dem Träger entfernt, insbesondere abgelöst.

Weiter bevorzugt wird das Substrat vor dem Durchführen von Schritt b) auf einen Kern unter Ausbildung eines zylindrischen, schlauchförmigen oder konisch gestalteten Substrats aufgebracht. Der Kern ist hierfür zweckmäßigerweise zylindrisch, schlauchförmig oder konisch gestaltet. Insbesondere kann der Kern stempelförmig gestaltet sein. Weiter kann der Kern ein Metall aufweisen oder aus einem Metall bestehen. Bei dem Metall kann es sich beispielsweise um Aluminium handeln.

Bevorzugt wird das zylindrische, schlauchförmige oder konisch gestaltete Substrat von dem Kern abgelöst und der Kern durch ein wasserlösliches Material, insbesondere ein wasserlösliches Wachs, ersetzt. Mit anderen Worten wird der bisherige Kern bevorzugt durch einen wasserlöslichen Kern, insbesondere aus einem wasserlöslichen Wachs, ersetzt. Das wasserlösliche Wachs kann einen Schmelzpunkt von 50 °C bis 60 °C, insbesondere 55 °C bis 60 °C, besitzen. Bevorzugt wird das Ablösen des zylindrischen, schlauchförmigen oder konisch gestalteten Substrats von dem Kern und/oder das Ersetzen des Kerns durch das wasserlösliche Material im Inneren eines Formwerkzeugs oder einer Gießform, insbesondere mehrteilig, bevorzugt zweiteilig, ausgebildeten und öffenbaren Formwerkzeugs bzw. Gießform, durchgeführt. Vorzugsweise weist das Formwerkzeug bzw. die Gießform, insbesondere in einem zusammengesetzten oder geschlossenen Zustand, eine Form auf, die der Form des herzustellenden Produkts entspricht.

Bevorzugt wird anschließend die wenigstens eine Mehrschichtstruktur auf das zylindrische, schlauchförmige oder konisch gestaltete Substrat transferiert bzw. übertragen. Dieser Schritt wird vorzugsweise außerhalb des Formwerkzeugs bzw. der Gießform durchgeführt.

Danach wird das wasserlösliche Material entfernt, insbesondere durch Spülung mit Wasser. Dieser Schritt kann wiederum bevorzugt in dem Formwerkzeug bzw. der Gießform durchgeführt werden. Dadurch können vorteilhafterweise unerwünschte Belastungen der wenigstens einen Mehrschichtstruktur vermieden werden.

Vorzugsweise wird die wenigstens eine Mehrschichtstruktur beim Durchführen von Schritt b) mithilfe der Transferfolie auf das Substrat aufgebracht. Vorzugsweise wird die Transferfolie nach Ausbilden einer Verbindung, vorzugsweise stoffschlüssigen Verbindung, zwischen der wenigstens einen Mehrschichtstruktur, insbesondere der wenigstens einen Elastomermaterialschicht und/oder wenigstens einen zusätzlichen Elastomermaterialschicht der wenigstens einen Mehrschichtstruktur, und dem Substrat entfernt. Die Verbindung kann beispielsweise durch Aushärtung oder Vernetzung des Elastomermaterials oder einer Vorstufe, insbesondere eines Prepolymers, davon erfolgen und insbesondere auf der Ausbildung kovalenter Bindungen beruhen. Alternativ oder in Kombination kann die Verbindung auf der Ausbildung von nichtkovalenten Bindungen, insbesondere ausgewählt aus der Gruppe bestehend aus Van-der-Waals-Kräften, Wasserstoffbrückenbindungen, ionischen Bindungen, koordinativen Bindungen und einer Kombination von wenigstens zwei der vorgenannten nichtkovalenten Bindungen beruhen. Weiter kann die Verbindung zwischen der wenigstens einen Mehrschichtstruktur, insbesondere der wenigstens einen Elastomermaterialschicht und/oder wenigstens einen zusätzlichen Elastomermaterialschicht der wenigstens einen Mehrschichtstruktur, und dem Substrat in einem Formwerkzeug oder einer Gießform, insbesondere in dem bereits erwähnten Formwerkzeug bzw. in der bereits erwähnten Gießform, ausgebildet werden. Hierzu kann das mit der wenigstens einen Mehrschichtstruktur versehene Substrat während eines Zeitraums von 12 h bis 72 h, insbesondere 24 h bis 48 h, und insbesondere bei einer Temperatur von 18 °C bis 30 °C, bevorzugt 20 °C bis 25 °C, in dem Formwerkzeug bzw. der Gießform verbleiben.

Weiter bevorzugt weist das Verfahren ferner einen Schritt c) auf:
- Anformen von zusätzlichem Elastomermaterial, insbesondere mittels Spritzgießen, an das mit der wenigstens einen Mehrschichtstruktur versehene Substrat, insbesondere die wenigstens eine auf das Substrat transferierte, d.h. übertragene, Mehrschichtstruktur.

Dadurch kann vorteilhafterweise eine finale, d.h. endgültige, Form des mit der wenigstens einen Mehrschichtstruktur versehenen Substrats und mithin des herzustellenden Produkts festgelegt werden.

Vorzugsweise wird der Schritt c) (ebenfalls) in einem Formwerkzeug oder einer Gießform, insbesondere in dem bereits erwähnten Formwerkzeug bzw. in der bereits erwähnten Gießform, durchgeführt.

Bezüglich weiterer Merkmale und Vorteile des Verfahrens wird vollständig auf die unter dem ersten Erfindungsaspekt gemachten Ausführungen Bezug genommen, die sinngemäß auch für das Verfahren gemäß zweitem Erfindungsaspekt gelten.

Weitere Merkmale und Vorteile der Erfindung ergeben sich aus den Ansprüchen sowie aus der nachfolgenden Beschreibung bevorzugter Ausführungsformen anhand von Figuren und Beispielen. Dabei können Merkmale der Erfindung jeweils für sich alleine oder in Kombination miteinander verwirklicht sein. Die nachfolgend beschriebenen Ausführungsformen dienen der weiteren Erläuterung der Erfindung, ohne diese hierauf zu beschränken.

### BEISPIELTEIL

### 1. Experimentelle Methoden

### 1.1 MPBC-Probendesign

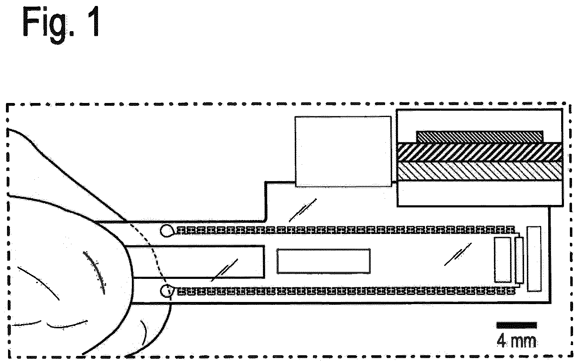

Es wurden Metall-Polymer-Mehrschicht-Komposite, insbesondere Metall-Polymer-Zweischicht-Komposite (metal-polymer bilayer composite, MPBC) mit einer dünnen (ca. 100 µm) Polydimethylsiloxan-Membran (PDMS-Membran) verwendet, die mit strukturierten 40 nm Au-Schichten funktionalisiert ist. Es wurden zwei verschiedene Strukturierungsmethoden verwendet, bei einer wurde eine herkömmliche fotolithographische Bearbeitung und bei der anderen eine Lochmaske eingesetzt. Die Au-Metallisierung ermöglicht dehnungsempfindliche Bereiche sowie Zuleitungsbereiche, die bei Belastung ihren Widerstand nur geringfügig verändern. Die Zuleitungen umfassen dabei selbstähnliche Serpentinenstrukturen. Der Sensorbereich nutzt eine dehnungsempfindliche Oberfläche mit Mikrorissen. Ein Beispiel einer funktionalisierten Metall-Polymer-Mehrschicht-Komposit-Struktur ist in Figur 1 gezeigt.

### 1.2 Substratvorbereitung

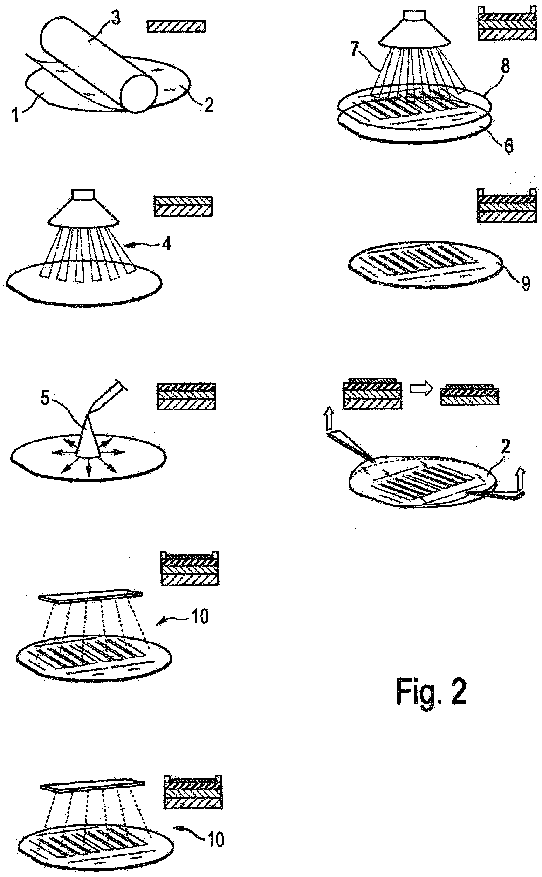

Die Hauptschritte des Prozesses sind in Figur 2 gezeigt. Es wurden einseitig polierte 4 Zoll Wafer 1 mit einer Dicke von 0,5 mm als Handling-Plattform verwendet. Eine UV-empfindliche Dicingfolie 2 (auch als Sägefolie bezeichnet) aus Polyethylenterephthalat (PET) (Adwill D-203, 50 µm) wurde mit einer Weichschaumrolle 3 auf den Wafer 1 übertragen. Die aufgebrachte Folie 2 wurde mit einem Skalpell entlang der Waferkanten geschnitten und anschließend mit UV-Licht 4 belichtet, um die Adhäsion an den Wafer 1 zu verringern (Spezialgerät mit 5 STER-L-RAY^{®} Niederdruck-Quecksilberlampen). Bei einer Minimaldosis von etwa 160 mJ/cm² verringert sich die Adhäsion auf etwa 1 % ihres ursprünglichen Werts (gemäß dem Datenblatt von 772,2 N/m auf 5,8 N/m). Um eine gleichmäßige und maximale Adhäsionsverringerung zu gewährleisten, erfolgte die UV-Aktivierung bei einer erhöhten Dosis von etwa 250 mJ/cm² und wurde unter einer Stickstoffatmosphäre durchgeführt, um eine Ozonbildung zu verhindern. Nach der UV-Behandlung wurde ein entgastes, blasenfreies PDMS-Prepolymer 5 (Sylgard 184^{®} Elastomer-Kit, Mischung 10:1 (Basis zu Härter), gemischt mit THINKY 250-ARE Planeten-Zentrifugalmischer) durch Rotationsbeschichtung auf den mit der Folie 2 bedeckten Handling-Wafer 1 bei 600 UpM (Beschleunigung 200 UpM /s) über 60 sec aufgebracht. Anschließend wurde ein Vakuum angelegt (10 min), um eine defektfreie und ebenmäßige Membran zu gewährleisten, bevor sie 30 min lang bei 90 °C in einem Ofen gehärtet wurde.

### 1.3 Strukturierung der PDMS-Membran

Im Fall der fotolithographischen Strukturierung wurde ein Negativ-Lift-Off-Fotolack 6 (AZ nLOF^{®} 2070, Merck KGaA) mittels Rotationsbeschichtung bei 3000 UpM (Beschleunigung 1000 UpM/s) über 60 sec direkt auf die PDMS-Oberfläche aufgebracht. Der Softbake bei 100 °C über 2 min auf einer Heizplatte führte zu einer Fotolackdicke von etwa 6,5 µm. Die Belichtung 7 mit einer Chrommaske 8 setzte einen SUSS MA6 Mask-Aligner (350 W Hochdruck-Quecksilberlampe) bei einer Dosis von 70 mJ/cm² ein. Das nachfolgende Backen (post exposure bake PEB) wurde auf einer Heizplatte 3 min lang bei 115 °C durchgeführt. Zur Vermeidung von Rissbildungen in der stabilisierten Fotolackschicht aufgrund von unterschiedlichen thermischen Expansionskoeffizienten der beteiligten Schichten in der Mehrschichtstruktur wurden die belichteten Wafer direkt in einen vorgeheizten Ofen von 100 °C überführt. Danach ließ man sie langsam über einen Zeitraum von etwa 5 h auf Raumtemperatur abkühlen. Zur Entwicklung wurde der AZ-Entwickler (Merck KGaA, unverdünnt, Hochgeschwindigkeitskonfiguration) in einem Laborglas auf einem analogen Orbitalschüttler (RS-OS 5 Phoenix-Instruments) vorbereitet. Bei leichter Fluid-Bewegung wurden die Strukturen 1 min 50 sec lang entwickelt und danach mit DI-Wasser abgespült und unter einem Stickstoffstrom getrocknet.

Im Falle einer Strukturierung der Metallschicht in einem Lochmaskenprozess wurde eine 50 µm Nickel-Schablonenmaske 9 (Stencil-Mask), hergestellt von der Firma applied microSWISS GmbH in einem geeigneten UV-LIGA-Prozess, verwendet. Da die Maske die äußeren Abmessungen eines Standard-Wafer von 4 Zoll aufwies, wurde die Mechanik des SUSS MA6 Mask-Aligners verwendet, um die Maske mittels Flat-Ausrichtung (Flat Alignment) und Magneten auf die PDMS-Membranoberfläche aufzubringen. Der oberflächengetreue Kontakt zwischen dem PDMS und der glatten Maskenoberfläche gewährleistete eine stabile Positionierung aufgrund von van-der-Waals-Wechselwirkungen ohne Notwendigkeit einer weiteren Fixierung.

### 1.4 PVD-Metallabscheidung und Ablösung

Die Gasphasenabscheidung 10 wurde mittels thermischer Verdampfung in einer Vakuumbeschichtungseinrichtung Edwards Auto 306 durchgeführt. Zur Verbesserung der Adhäsion der 40 nm Au-Deckschicht wurde zuvor eine Zwischenschicht aus Ti von 4 nm abgeschieden, ohne dazwischen das Vakuum (etwa 2*10⁻⁶ mbar) zu unterbrechen. Nach dem Beschichtungsverfahren wurde entweder der Fotolack in Aceton mit leichter Fluidanregung gelöst und das resultierende Mehrschichtsystem wurde danach mit DI-Wasser gespült, oder die Lochmaske wurde sanft von der PDMS-Oberfläche abgezogen. Anschließend konnte die UVaktivierte stabilisierende Transferfolie 2 leicht vom Wafer 1 gelöst werden, ohne relevante Belastungen auf die dünne funktionalisierte PDMS-Membran 6 auszuüben.

### 2. Übertragungsverfahren

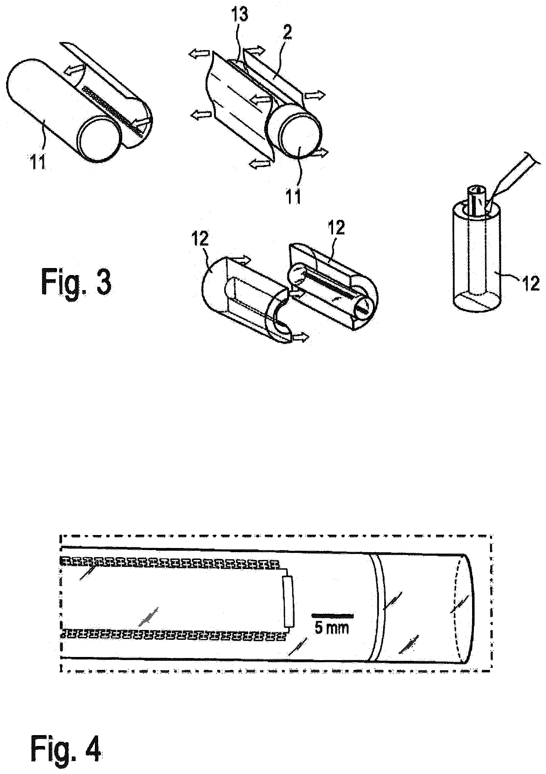

In Figur 3 ist der Übertragungsprozess auf ein zylinderförmiges PDMS-Basissubstrat 11 gezeigt. Die zylinderförmige Basis 11 war in einer Spezialform aus Polymethylmethacrylat (PMMA) mit einem konstanten Außendurchmesser von 10 mm gegossen. Die Wanddicke betrug größtenteils 400 µm und an spezifizierten Bereichen nur 200 µm. Da die angegebene Übertragung eine Verbindung von PDMS zu PDMS über teilweises Härten verwendete, war es in der Praxis zweckmäßig, die dazwischenliegende PDMS-Verbindungsschicht durch Rotationsbeschichtung vor dem Ablösen der Folie aufzubringen. Eine PDMS-PDMS-Verbindung über teilweises Härten ermöglichte hohe Verbindungsfestigkeiten, während es leicht auszuführen ist. Hier wurde eine dünne ungefähr 60 µm dicke PDMS-Schicht durch Rotationsbeschichtung auf die Oberseite der strukturierten Schicht bei 900 rpm über 60 s (Beschleunigung 200 UpM/s) aufgetragen und danach 45 min lang bei 50 °C gehärtet. Dies führte zu einer Stabilisierung des PDMS-Films, während er klebrige Eigenschaften beibehielt, die mit einer dazwischenliegenden Klebstoffschicht vergleichbar waren. Anschließend wurde der betreffende Probenteil mit einem Skalpell vorgeschnitten, die stabilisierende Transferfolie 2 wurde mit einer Pinzette vom Handling-Wafer abgelöst (siehe Schritt 4 in Figur 2) und die teilweise gehärtete PDMS-Fläche wurde an der geeigneten Position an der zylinderförmigen Basis 11 angebracht (Schritt 1 in Figur 3). Das Probestück wurde dann in eine zylinderförmige PMMA-Form 12 eingesetzt und 48 h lang bei Raumtemperatur darin belassen, so dass sie vollständig aushärtete. Als letzte Schritte wurde die Transferfolie 2 von der verbundenen Membranschicht 13 abgelöst (Figur 3, Schritt 2), und es wurden ein weiterer Gießprozess (Figur 3, Schritte 3 + 4) und ein anschließendes Härten bei Raumtemperatur über 48 h durchgeführt, um einen gleichmäßigen zylinderförmigen Sensor zu erhalten.

### 3. Charakterisierung und Ergebnisse

Um zu gewährleisten, dass die übertragene Metallmorphologie nicht durch undefinierte Belastungen nach dem letzten Schritt verändert wird, war es notwendig, den fertigen PDMS-Zylinder sorgfältig aus der Form zu entnehmen. Deshalb wurde der innere Teil der Gießform, die die veränderliche Wanddicke des PDMS-Basiszylinders definierte, vor dem Übertragungsprozess durch ein wasserlösliches Klebewachs ersetzt (2-M19 soluble stic wax, Paramelt B.V.). Dies erforderte, dass die anschließenden Härtungsschritte des Verbindungsprozesses und die finalen Gießschritte bei niedriger Temperatur durchgeführt wurden, um ein Schmelzen des Wachses zu vermeiden (Tropfschmelzpunkt ca. 57 °C).

Figur 4 zeigt den fertigen PDMS-Zylinder mit den integrierten Sensorstrukturen. Als Beweis des Anspruchs, dass der Prozess die Handhabung und Übertragung von fragilen Oberflächen-Morphologien ermöglicht, wurden verschiedene Oberflächen (mit Mikrorissen und welligen periodischen Strukturen) durch selektive Plasmavorbehandlung sowie als Variation der Abscheidungsrate eingeführt. Zur Charakterisierung der Morphologie im Anfangszustand vor der Verbindung, nach dem Ablösen des Handling-Wafers und im Endzustand an der zylinderförmigen Basis, wurde dieselbe Struktur bei jedem Schritt in einem SEM (Philips XL30) untersucht. Zur Charakterisierung der Morphologie nach der Übertragung zur zylinderförmigen Basis wurde eine Spezialhalterung aus Aluminium, die die Wölbung der PDMS-Basis simuliert, verwendet.

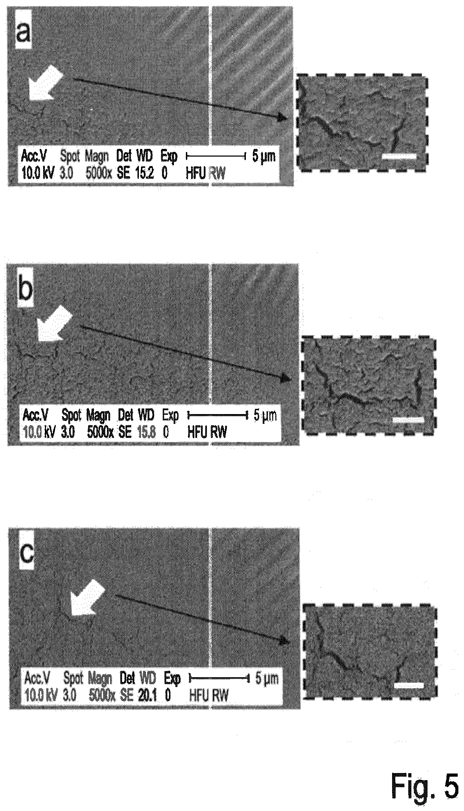

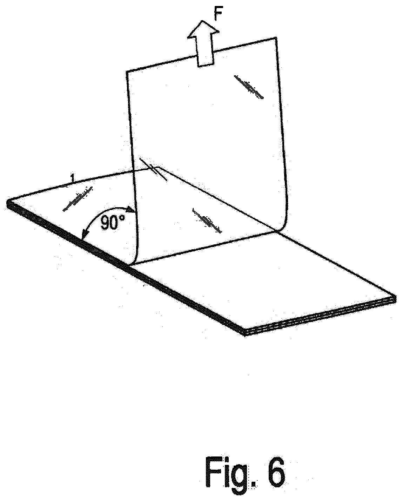

In Figur 5 ist ein ausgewählter Morphologiebereich des vorbereiteten Probestücks in jedem relevanten Schritt der vorgestellten Verfahrensweise gezeigt. Wie zu erkennen ist, veränderte sich die fragile Rissmorphologie während der Handhabung der Membran nicht und die einzige Veränderung betraf die leichte Kompression aufgrund der Wölbung der zylinderförmigen Basis (Figur 5 c), während weder eine Delaminierung noch eine Risserweiterung zu beobachten war. Weiterhin haben einfache Zweileitermessungen des Widerstandes gezeigt, dass die elektrische Integrität der Struktur bei etwa 4 kΩ erhalten blieb.

Da es verschiedene Möglichkeiten für das Verbindungsverfahren von Membran an Substrat gibt, wurde eine 90°-Schälprüfung (siehe DIN EN 28510-1:2014) durchgeführt, um die notwendige Abschälkraft zum Ablösen der Abdeckfolie von der PDMS-Membran zu ermitteln (Schritt 2, Figur 3). Zu diesem Zweck wurden Probestücke mit einer PDMS-Membran von 100 µm, die gemäß dem oben vorgestellten Substratvorbereitungsverfahren hergestellt wurden (Schritt 1, Figur 2), vom Handling-Wafer abgelöst und die PDMS-Oberfläche mit der polierten Seite von vorgeschnittenen Waferstücken (30 mm breit) nach Aktivierung in einem Sauerstoffplasma (30 sec bei 100 W) verbunden. Dann wurde die Transferfolie an der Lastzelle der Abschäl-Prüfeinrichtung angebracht und die Tests wurden bei einer konstanten Geschwindigkeit von 50 mm/min bei einem Abschälwinkel von 90° durchgeführt. Da die Untersuchungen durch die Größe der Wafer und die Breite der Flats begrenzt waren (Wafer wurden senkrecht zum Hauptflat nahe den Kanten des Flats geschnitten), betrug die maximale Abschällänge 90 mm und die verwendete Abschälbreite betrug 20 mm. Insgesamt 5 Proben wurden zwischen 20 mm und 80 mm Abschällänge geprüft und analysiert, um Kanteneffekte auszuschließen. Die mittlere Abschälkraft wurde mit 0,07 N/mm bestimmt (SD 0,011 N/mm).

| **Nr.** | **Abschälkraft [N/mm]** |
|---|---|
| 1. | **0.076** |
| 2. | **0.076** |
| 3. | **0.082** |
| 4. | **0.078** |
| 5. | **0.051** |
| Ø | **0.07** |
| Standardabweichung | **0.01** |

## Patentansprüche

1. Medizinprodukt mit wenigstens einer Mehrschichtstruktur, die nachfolgende übereinanderliegend angeordnete Schichten aufweist:
- wenigstens eine Metallschicht und
- wenigstens eine Elastomermaterialschicht,
**dadurch gekennzeichnet, dass** die wenigstens eine Mehrschichtstruktur eine Sensoreinheit des Medizinprodukts definiert, wobei das Medizinprodukt als Ballonkatheter gestaltet ist und der Ballon des Katheters die wenigstens eine Mehrschichtstruktur aufweist.

2. Medizinprodukt nach Anspruch 1, **dadurch gekennzeichnet, dass** die wenigstens eine Elastomermaterialschicht ein Elastomermaterial, insbesondere ein hyperelastisches Material, aufweist oder aus einem Elastomermaterial, insbesondere hyperelastischen Material, besteht, das ausgewählt ist aus der Gruppe bestehend aus Elastomere, thermoplastische Elastomere, thermoplastische Polyamidelastomere, thermoplastische Copolyesterelastomere, thermoplastische Elastomere auf Olefinbasis, thermoplastische Styrol-Blockcopolymere, thermoplastische Elastomere auf Urethanbasis, thermoplastische Vulkanisate auf Olefinbasis, vernetzte thermoplastische Elastomere auf Olefinbasis, Vulkanisate von Naturkautschuken, Vulkanisate von Synthesekautschuken, Styrol-Butadien-Kautschuk, Butadien-Kautschuk (BR), AcrylnitrilButadien-Kautschuk (NBR), Butylkautschuk (IIR), Ethylen-Propylen-Dien-Kautschuk (EPDM), Chloropren-Kautschuk (CR), Polyisoprenkautschuk (IR), Polyalkylsiloxane, Polydimethylsiloxan, Silikonkautschuke, Silikonelastomere, Methyl-Silikon, Vinyl-Methyl-Silikon, Phenyl-Vinyl-Methyl-Silikon, Phenyl-modifiziertes Silikon, Fluoroalkyl-Silikon, Fluor-Vinyl-Methyl-Silikon und Mischungen von wenigstens zwei der vorgenannten Elastomermaterialien.

3. Medizinprodukt nach Anspruch 1 oder 2, **dadurch gekennzeichnet, dass** die wenigstens eine Metallschicht wenigstens ein Metall, insbesondere in elementarer Form oder in Form einer Legierung, aufweist oder aus wenigstens einem Metall, insbesondere in elementarer Form oder in Form einer Legierung, besteht, das ausgewählt ist aus der Gruppe bestehend aus Gold, Platin, Indium, Zinn, Kupfer, Silber, Gallium und Legierungen von wenigstens zwei der vorgenannten Metalle.

4. Medizinprodukt nach einem der vorhergehenden Ansprüche, **dadurch gekennzeichnet, dass** die wenigstens eine Elastomermaterialschicht eine Schichtdicke von 0,0001 mm bis 0,2 mm, insbesondere 0,0005 mm bis 0,1 mm, vorzugsweise 0,001 mm bis 0,05 mm, und die wenigstens eine Metallschicht eine Schichtdicke von ≤ 150 nm, insbesondere 10 nm bis 100 nm, vorzugsweise 40 nm bis 80 nm, aufweist.

5. Medizinprodukt nach einem der vorhergehenden Ansprüche, **dadurch gekennzeichnet, dass** die wenigstens eine Metallschicht die wenigstens eine Elastomermaterialschicht unmittelbar bedeckt.

6. Medizinprodukt nach einem der vorhergehenden Ansprüche, **dadurch gekennzeichnet, dass** zwischen der wenigstens einen Metallschicht und der wenigstens einen Elastomermaterialschicht wenigstens eine Adhäsionsschicht ausgebildet ist.

7. Medizinprodukt nach Anspruch 6, **dadurch gekennzeichnet, dass** die wenigstens eine Metallschicht die wenigstens eine Adhäsionsschicht unmittelbar und vollständig und die wenigstens eine Adhäsionsschicht die wenigstens eine Elastomermaterialschicht unmittelbar und nur bereichsweise bedeckt.

8. Medizinprodukt nach Anspruch 6 oder 7, **dadurch gekennzeichnet, dass** die wenigstens eine Adhäsionsschicht wenigstens ein Material aufweist oder aus wenigstens einem Material besteht, das ausgewählt ist aus der Gruppe bestehend aus Titan, Aluminium, Chrom und Mischungen von wenigstens zwei der vorgenannten Materialien.

9. Medizinprodukt nach einem der vorhergehenden Ansprüche, **dadurch gekennzeichnet, dass** die wenigstens eine Mehrschichtstruktur ferner wenigstens eine zusätzliche Elastomermaterialschicht aufweist, die die wenigstens eine Metallschicht unmittelbar bedeckt.

10. Medizinprodukt nach einem der vorhergehenden Ansprüche, **dadurch gekennzeichnet, dass** die wenigstens eine Mehrschichtstruktur oder die wenigstens eine Metallschicht eine variierende Oberflächenmorphologie, insbesondere Oberflächenbereiche mit Rissen, insbesondere Mikrorissen, und Oberflächenbereiche, insbesondere wellige Oberflächenbereiche, ohne Risse, aufweist.

11. Medizinprodukt nach einem der vorhergehenden Ansprüche, **dadurch gekennzeichnet, dass** die wenigstens eine Mehrschichtstruktur Teil einer Oberfläche des Medizinprodukts bildet oder in einer Wandung des Medizinprodukts integriert ist.

12. Verfahren zum Herstellen eines Medizinprodukts nach einem der vorhergehenden Ansprüche, aufweisend die nachfolgenden Schritte:
a) Herstellen wenigstens einer Mehrschichtstruktur mit wenigstens einer Metallschicht und wenigstens einer Elastomermaterialschicht und
b) Transferieren der wenigstens einen Mehrschichtstruktur auf ein Substrat unter Ausbildung eines mit der wenigstens einen Mehrschichtstruktur versehenen Substrats,
wobei die wenigstens eine Mehrschichtstruktur eine Sensoreinheit des Medizinprodukts definiert, wobei das Medizinprodukt als Ballonkatheter gestaltet ist und der Ballon des Katheters die wenigstens eine Mehrschichtstruktur aufweist.

## Claims

1. A medical device having at least one multilayer structure that includes the following layers arranged on top of one another:
- at least one metal layer and
- at least one elastomer material layer,
**characterized in that** the at least one multilayer structure defines a sensor unit of the medical device, wherein the medical device takes the form of a balloon catheter and the balloon of the catheter includes the at least one multilayer structure.

2. The medical device as claimed in claim 1, **characterized in that** the at least one elastomer material layer includes an elastomer material, more particularly a hyperelastic material, or consists of an elastomer material, more particularly hyperelastic material, selected from the group consisting of elastomers, thermoplastic elastomers, thermoplastic polyamide elastomers, thermoplastic copolyester elastomers, olefin-based thermoplastic elastomers, thermoplastic styrene block copolymers, urethane-based thermoplastic elastomers, olefin-based thermoplastic vulcanizates, olefin-based crosslinked thermoplastic elastomers, natural rubber vulcanizates, synthetic rubber vulcanizates, styrene-butadiene rubber, butadiene rubber (BR), acrylonitrile-butadiene rubber (NBR), butyl rubber (IIR), ethylene-propylene-diene rubber (EPDM), chloroprene rubber (CR), polyisoprene rubber (IR), polyalkylsiloxanes, polydimethylsiloxane, silicone rubbers, silicone elastomers, methyl silicone, vinyl methyl silicone, phenyl vinyl methyl silicone, phenyl-modified silicone, fluoroalkyl silicone, fluoro vinyl methyl silicone, and mixtures of at least two of the aforementioned elastomer materials.

3. The medical device as claimed in claim 1 or 2, **characterized in that** the at least one metal layer includes at least one metal, more particularly in elemental form or in the form of an alloy, or consists of at least one metal, more particularly in elemental form or in the form of an alloy, selected from the group consisting of gold, platinum, indium, tin, copper, silver, gallium and alloys of at least two of the aforementioned metals.

4. The medical device as claimed in any of the preceding claims, **characterized in that** the at least one elastomer material layer has a layer thickness of 0.0001 mm to 0.2 mm, more particularly 0.0005 mm to 0.1 mm, preferably 0.001 mm to 0.05 mm, and the at least one metal layer has a layer thickness of ≤ 150 nm, more particularly 10 nm to 100 nm, preferably 40 nm to 80 nm.

5. The medical device as claimed in any of the preceding claims, **characterized in that** the at least one metal layer directly covers the at least one elastomer material layer.

6. The medical device as claimed in any of the preceding claims, **characterized in that** at least one adhesion layer is formed between the at least one metal layer and the at least one elastomer material layer.

7. The medical device as claimed in claim 6, **characterized in that** the at least one metal layer covers the at least one adhesion layer directly and completely and the at least one adhesion layer covers the at least one elastomer material layer directly and only in regions.

8. The medical device as claimed in claim 6 or 7, **characterized in that** the at least one adhesion layer includes at least one material or consists of at least one material selected from the group consisting of titanium, aluminum, chromium, and mixtures of at least two of the aforementioned materials.

9. The medical device as claimed in any of the preceding claims, **characterized in that** the at least one multilayer structure further includes at least one additional elastomer material layer that directly covers the at least one metal layer.

10. The medical device as claimed in any of the preceding claims, **characterized in that** the at least one multilayer structure or the at least one metal layer has a varying surface morphology, more particularly surface regions with cracks, more particularly microcracks, and surface regions, more particularly corrugated surface regions, without cracks.

11. The medical device as claimed in any of the preceding claims, **characterized in that** the at least one multilayer structure forms part of a surface of the medical device or is integrated in a wall of the medical device.

12. A process for producing a medical device according to any of the preceding claims, including the following steps:
a) producing at least one multilayer structure having at least one metal layer and at least one elastomer material layer and
b) transferring the at least one multilayer structure to a substrate, with the formation of a substrate provided with the at least one multilayer structure,
wherein the at least one multilayer structure defines a sensor unit of the medical device, wherein the medical device takes the form of a balloon catheter and the balloon of the catheter includes the at least one multilayer structure.

## Revendications

1. Dispositif médical comportant au moins une structure multicouche qui comporte les couches suivantes disposées les unes au-dessus des autres :
- au moins une couche métallique et
- au moins une couche de matériau élastomère,
**caractérisé en ce que** ladite au moins une structure multicouche définit une unité de détection du dispositif médical, le dispositif médical étant configuré sous forme de cathéter à ballonnet et le ballonnet du cathéter comportant ladite au moins une structure multicouche.

2. Dispositif médical selon la revendication 1, **caractérisé en ce que** ladite au moins une couche de matériau élastomère comporte un matériau élastomère, en particulier un matériau hyperélastique, ou est constituée d'un matériau élastomère, en particulier d'un matériau hyperélastique, choisi dans le groupe constitué par des élastomères, des élastomères thermoplastiques, des élastomères polyamides thermoplastiques, des élastomères copolyesters thermoplastiques, des élastomères thermoplastiques à base d'oléfines, des copolymères séquencés de styrène thermoplastiques, des élastomères thermoplastiques à base d'uréthane, des vulcanisats thermoplastiques à base d'oléfine, des élastomères thermoplastiques réticulés à base d'oléfine, des vulcanisats de caoutchoucs naturels, des vulcanisats de caoutchoucs synthétiques, un caoutchouc de styrène-butadiène, un caoutchouc de butadiène (BR), un caoutchouc d'acrylonitrile-butadiène (NBR), un caoutchouc de butyle (IIR), un caoutchouc d'éthylène-propylène-diène (EPDM), un caoutchouc de chloroprène (CR), un caoutchouc de polyisoprène (IR), des polyalkylsiloxanes, un polydiméthylsiloxane, des caoutchoucs de silicone, des élastomères de silicone, la méthylsilicone, la vinyl-méthyl-silicone, la phényl-vinyl-méthyl-silicone, une silicone modifiée par un phényle, une fluoroalkyl-silicone, une fluorovinyl-méthyl-silicone et des mélanges d'au moins deux des matériaux élastomères précités.

3. Dispositif médical selon la revendication 1 ou 2, **caractérisé en ce que** la ou les couches métalliques comprennent au moins un métal, notamment sous forme élémentaire ou sous forme d'alliage, ou sont constituées d'au moins un métal, notamment sous forme élémentaire ou sous forme d'alliage, qui est choisi dans le groupe constitué par l'or, le platine, l'indium, l'étain, le cuivre, l'argent, le gallium et des alliages d'au moins deux des métaux précités.

4. Dispositif médical selon l'une quelconque des revendications précédentes, **caractérisé en ce que** ladite au moins une couche de matériau élastomère présente une épaisseur de couche de 0,0001 mm à 0,2 mm, en particulier de 0,0005 mm à 0,1 mm, de préférence de 0,001 mm à 0,05 mm, et **en ce que** ladite au moins une couche métallique présente une épaisseur de couche ≤ 150 nm, en particulier de 10 nm à 100 nm, de préférence de 40 nm à 80 nm.

5. Dispositif médical selon l'une quelconque des revendications précédentes, **caractérisé en ce que** ladite au moins une couche métallique recouvre directement ladite au moins une couche de matériau élastomère.

6. Dispositif médical selon l'une quelconque des revendications précédentes, **caractérisé en ce qu'**au moins une couche d'adhérence est formée entre ladite au moins une couche métallique et ladite au moins une couche de matériau élastomère.

7. Dispositif médical selon la revendication 6, **caractérisé en ce que** ladite au moins une couche métallique recouvre directement et entièrement ladite au moins une couche d'adhérence et **en ce que** ladite au moins une couche d'adhérence recouvre directement et seulement par zones ladite au moins une couche de matériau élastomère.

8. Dispositif médical selon la revendication 6 ou 7, **caractérisé en ce que** ladite au moins une couche adhésive comprend au moins un matériau ou est constituée d'au moins un matériau choisi dans le groupe constitué par le titane, l'aluminium, le chrome et des mélanges d'au moins deux des matériaux précités.

9. Dispositif médical selon l'une quelconque des revendications précédentes, **caractérisé en ce que** ladite au moins une structure multicouche comprend en outre au moins une couche supplémentaire de matériau élastomère qui recouvre directement ladite au moins une couche métallique.

10. Dispositif médical selon l'une quelconque des revendications précédentes, **caractérisé en ce que** ladite au moins une structure multicouche ou ladite au moins une couche métallique présente une morphologie de surface différente, en particulier des zones de surface ayant des fissures, en particulier des microfissures, et des zones de surface, en particulier des zones de surface ondulées, sans fissures.

11. Dispositif médical selon l'une quelconque des revendications précédentes, **caractérisé en ce que** ladite au moins une structure multicouche fait partie d'une surface du dispositif médical ou est intégrée dans une paroi du dispositif médical.

12. Procédé de fabrication d'un dispositif médical selon l'une quelconque des revendications précédentes, comprenant les étapes suivantes :
a) réalisation d'au moins une structure multicouche comportant au moins une couche métallique et au moins une couche de matériau élastomère et
b) transférer de ladite au moins une structure multicouche sur un substrat pour former un substrat pourvu de ladite au moins une structure multicouche,
ladite au moins une structure multicouche définissant une unité de détection du dispositif médical, le dispositif médical étant configuré sous forme de cathéter à ballonnet et le ballonnet du cathéter comportant ladite au moins une structure multicouche.
